(19) Europäisches Patentamt
European Patent Office
Office européen des brevets

(11) Veröffentlichungsnummer: **0 144 283**
**A1**

(12) # EUROPÄISCHE PATENTANMELDUNG

(21) Anmeldenummer: 84810470.9

(22) Anmeldetag: 28.09.84

(51) Int. Cl.⁴: **A 01 N 47/36**
A 01 N 25/32

(30) Priorität: 04.10.83 CH 5389/83

(43) Veröffentlichungstag der Anmeldung: 12.06.85 Patentblatt 85/24

(84) Benannte Vertragsstaaten: BE CH DE FR GB IT LI NL

(71) Anmelder: **CIBA-GEIGY AG**
Postfach
CH-4002 Basel(CH)

(72) Erfinder: **Gerber, Hans-Rudolf, Dr.**
Blözenweg 49
CH-4133 Pratteln(CH)

(72) Erfinder: **Bellucci, Sergio, Dr.**
Kapellstrasse 121
CH-4323 Wallbach(CH)

(54) **Herbizides Mittel.**

(57) Es werden Herbizide Mittel beschrieben, welche neben einem

a) herbizid wirksamen Sulfonylharnstoff der Formel

$Q-SO_2-NH-CO-NH-T$ (I)

worin Q ein substituierter Phenylrest der Formel

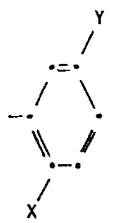

ist, worin X Halogen, Halogenalkenyl, Alkoxycarbonyl, Alkenyloxy, Halogenalkoxy, Alkoxyalkoxy, Nitro, Y Wasserstoff oder Halogen bedeutet, T ein substituierter sechsgliedriger heterocyclischer Rest der Formel

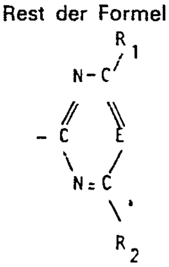

ist, worin E $-CH=$ oder $-N=$, $R_1$ Alkyl, Alkoxy, Halogenalkoxy, $R_2$ Alkyl, Alkoxy bedeutet,

b) einen antidotisch wirksamen Oximäther der Formel

$$Ar - (SO_n)_m - \underset{\underset{N - O - BA}{\|}}{C} - X$$

worin Ar einen gegebenenfalls substituierten Phenyl-, Naphthyl- oder einen 5-6 gliedrigen aromatischen Heterocyclus mit 1-3 Heteroatomen,

A Wasserstoff, einen $C_1$-$C_4$-Alkoxy-, $C_2$-$C_4$-Alkenyloxy-, $C_1$-$C_4$-Alkylthio-, $C_2$-$C_4$-Alkenylthio-, Carboxyl-, Carbamoyl-, $C_1$-$C_4$-Alkylcarbonyl-, $C_1$-$C_4$-Alkylcarbamoyl-, $C_1$-$C_4$-Alkoxycarbonyl-, Benzoyl und Carbanilidorest, wobei die Phenylringe unsubstituiert oder durch Halogen, Cyan, Nitro, $C_1$-$C_4$-Alkyl, $C_1$-$C_4$-Alkoxy oder $C_1$-$C_4$-Haloalkoxy substituiert sein können, sowie einen Furylcarbonyl-, Thiophencarbonyl- oder einen über das Stickstoff gebundenen Imidorest einer Dicarbonsäure,

B die direkte Bindung oder einen 1-4 gliedrigen Alkylen- oder Alkenylenrest, der geradkettig oder verzweigt sein kann,

X Wasserstoff, Halogen, einen Cyan-, $C_1$-$C_4$-Alkyl-, Carboxyl-, Carbamoyl- oder $C_1$-$C_4$-Alkylcarbamoylrest,

m Null oder die Zahl 1 und

n Null oder die Zahl 1 oder 2 bedeuten

enthalten, sowie die Verwendung dieser Mittel zur selektiven Unkrautbekämpfung.

EP 0 144 283 A1

CIBA-GEIGY AG
Basel (Schweiz)

5-14602/=

# Herbizides Mittel

Die Erfindung betrifft ein selektiv wirkendes herbizides Mittel, welches als herbiziden Wirkstoff einen Sulfonylharnstoff enthält zusammen mit einem antagonistisch wirkenden Oximäther (Antidote) welcher die Toleranz der Kulturpflanzen gegenüber der phytotoxischen Wirkung erhöht, ohne dass deswegen die Herbizidwirkung gegenüber den Unkräutern merklich abfällt. Die Erfindung betrifft ebenfalls die Verwendung dieses Mittels zur Unkrautbekämpfung in Nutzpflanzenkulturen.

Sulfonylharnstoffe mit Herbizidwirkung sind bekannt, siehe beispielsweise die US Patentschriften No. 4 127 405, 4 169 719, 4 238 628 und die offengelegten Europäischen Patentanmeldungen EP-A No 44 807 und 44 820.

Gegenüber anderen Herbiziden zeichnen sich diese Sulfonylharnstoffe durch ihre starke Wirkung aus. Aus ökologischer und ökonomischer Sicht ist die Verwendung kleiner Wirkstoffmengen vorteilhaft. Es bringt jedoch auch die Gefahr schädigender Ueberdosierung mit sich. Schadwirkungen sind nicht nur auf Ueberdosierung zurückzuführen, sie können auch auftreten wegen abnormalen klimatischen Verhältnissen oder wegen der Vorbehandlung des Bodens.

Es hat sich deshalb als wünschenswert erwiesen die Resistenz oder Toleranz der Kulturpflanzen gegenüber der phytotoxischen Wirkung der Sulfonylharnstoff-Herbizide zu erhöhen. Im US Patent No. 4 343 649 wird beschrieben, dass die bekannten Herbizid-Antidoten 1.8-Naphthalinsäureanhydrid, N,N-Diallyldichloracetamid sowie α-Cyano-methoximino-2-phenylacetonitril diese Aufgabe erfüllen, wenn man sie in Nutzpflanzenkulturen gleichzeitig mit dem Sulfonylharnstoff-Herbizid verwendet.

Ueberraschenderweise wurde nun gefunden, dass sich die Toleranz von Kulturpflanzen gegenüber der phytotoxischen Wirkung der Sulfonylharnstoff-Herbizide erhöht, ohne dass dadurch die Herbizidwirkung auf die Grosszahl der Unkräuter und Ungräser beeinträchtigt wird, wenn man die Sulfonylharnstoff-Herbizide zusammen mit Oximäthern verwendet welche als Herbizid-Antagonist oder Antidot wirken.

Die Sulfonylharnstoff-Herbizide entsprechen der allgemeinen Formel I

$$Q\text{-}SO_2\text{-}NH\text{-}CO\text{-}NH\text{-}T \qquad (I)$$

worin Q ein substituierter Phenylrest der Formel

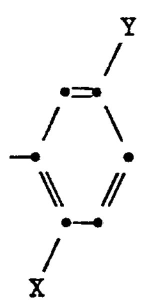

ist, worin X Halogen, $C_1$-$C_3$ Halogenalkenyl, $C_1$-$C_3$ Alkoxycarbonyl, $C_3$-$C_4$ Alkenyloxy, $C_1$-$C_3$ Halogenalkoxy, $C_1$-$C_3$ Alkoxy-$C_1$-$C_2$-alkoxy, Nitro, Y Wasserstoff oder Halogen bedeutet, T ein substituierter sechsgliedriger heterocyclischer Rest der Formel

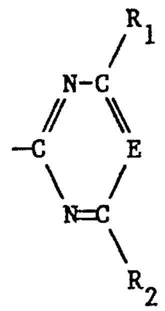

ist, worin E -CH= oder -N=, $R_1$ $C_1$-$C_3$ Alkyl, $C_1$-$C_3$ Alkoxy, $C_1$-$C_3$ Halogenalkoxy, $R_2$ $C_1$-$C_3$ Alkyl, $C_1$-$C_3$ Alkoxy bedeutet.

Verbindungen, worin Y = H ist, werden dabei bevorzugt.

- Unter Halogen kann z.B. Fluor, Chlor und Brom, insbesondere aber Chlor verstanden werden.
- Unter Halogenalkenyl kann z.B. Halogenäthenyl, Halogenpropenyl und bevorzugt 3,3,3-Trifluorpropenyl verstanden werden.
- Unter Alkoxycarbonyl kann z.B. Methoxy, Aethoxy, Propoxy oder Isopropoxycarbonyl und bevorzugt Methoxycarbonyl verstanden werden.
- Unter Alkenyloxy kann z.B. Allyloxy, Propenyloxy, Buten-2-yl-oxy, Buten-3-yl-oxy, 1-Methyl-alkyloxy, 2-Methylalkyloxy, 1-Methyl-propenyloxy und 3-Methyl-propenyloxy, insbesondere aber Allyloxy und Buten-2-yl-oxy verstanden werden.
- Unter Halogenalkoxy kann z.B. Halogenmethoxy, Halogenäthoxy, Halogenpropoxy und Halogenisopropoxy, bevorzugt aber Chlormethoxy, Chloräthoxy, Fluormethoxy, Fluoräthoxy, insbesondere bevorzugt als X 2-Chloräthoxy und Pentafluoräthoxy, als $R_1$ Difluormethoxy verstanden werden.
- Unter Alkoxy-alkoxy kann z.B. Methoxy-methoxy, 2-Methoxy-äthoxy, Aethoxy-methoxy, 2-Aethoxy-äthoxy, n-Propoxy-methoxy, Isopropoxymethoxy, 2-n-Propoxy-äthoxy, 2-Isopropoxy-äthoxy, insbesondere aber 2-Methoxy-äthoxy verstanden werden.

- Unter Y kann z.B. Wasserstoff oder Halogen, wie Fluor, Chlor, Brom, insbesondere Wasserstoff oder Chlor verstanden werden.
- Unter Alkyl kann z.B. Methyl, Aethyl, n-Propyl, i-Propyl, bevorzugt aber Methyl verstanden werden.
- Unter Alkoxy kann z.B. Methoxy, Aethoxy, n-Propoxy, i-Propoxy, bevorzugt aber Methoxy und Aethoxy verstanden werden.

Als herbizid besonders wirksame Sulfonylharnstoffe erweisen sich die Verbindungen der Formel I, worin Y = H ist.

Folgende Sulfonylharnstoffe werden als Herbizide insbesondere bevorzugt:
N-[2-(2'-Chloräthoxy)-phenyl-sulfonyl]-N'-(4-methyl-6-methoxy-triazin-2-yl)-harnstoff, N-[2-(2'-Methoxyäthoxy)-phenyl-sulfonyl]-N'-(4-methyl-6-methoxy-triazin-2-yl)-harnstoff, N-[2-(2-Butenyloxy)-phenyl-sulfonyl]-N'-(4-methyl-6-methoxy-triazin-2-yl)-harnstoff, N-[2-(3-Trifluor-propen-1-yl)-phenyl-sulfonyl]-N'-(4-methyl-6-methoxy-triazin-2-yl)-harnstoff, N-(2,5-Dichlorphenyl-sulfonyl)-N'-(4-methyl-6-methoxypyrimidin-2-yl)-harnstoff, N-(2-Methoxycarbonyl-phenyl-sulfonyl)-N'-(4-methyl-6-difluormethoxy-pyrimidin-2-yl)-harnstoff, N-(2-Pentafluoräthoxyphenyl-sulfonyl)-N'-(4,6-dimethoxy-triazin-2-yl)-harnstoff, N-(2-Chlorphenyl-sulfonyl)-N'-(4-methyl-6-methoxy-triazin-2-yl)-harnstoff, N-(2-Methoxycarbonyl-phenyl-sulfonyl)-N'-(4,6-dimethyl-pyrimidin-2-yl)-harnstoff, N-(2-Allyloxy-phenyl-sulfonyl)-N'-(4-methyl-6-äthoxy-triazin-2-yl)-harnstoff N-(2-Nitrophenyl-sulfonyl)-N'-(4-methyl-6-difluormethoxy-pyrimidin-2-yl)harnstoff und N-(2-Methoxycarbonyl-phenyl-sulfonyl)-N'-(4-methyl-6-methoxy-triazin-2-yl)-harnstoff.

Als Antidote zu diesen Sulfonylharnstoffen verwendet man einen Oximäther entsprechend der allgemeinen Formel II

$$\begin{array}{l} Ar-(SO_n)_m - \underset{\underset{\displaystyle N - O - B - A}{\|}}{C} - X \end{array} \qquad (II),$$

worin Ar einen gegebenenfalls substituierten Phenyl-, Naphthyl- oder einen 5-6-gliedrigen aromatischen oder benzannelierten Heterocyclus mit 1-3 Heteroatomen,

A Wasserstoff, einen $C_1$-$C_4$-Alkoxy, $C_2$-$C_4$-Alkenyloxy, $C_1$-$C_4$-Alkylthio-, $C_2$-$C_4$-Alkenylthio, Carboxyl-, Carbamoyl-, $C_1$-$C_4$-Alkylcarbonyl-, $C_1$-$C_4$-Alkylcarbamoyl-, $C_1$-$C_4$-Alkoxycarbonyl-, Benzoyl- und Carbanilidorest-, wobei die Phenylringe unsubstituiert oder durch Halogen, Cyan, Nitro, $C_1$-$C_4$-Alkyl, $C_1$-$C_4$-Alkoxy oder $C_1$-$C_4$-Haloalkoxy substituiert sein können, sowie einen Furylcarbonyl, Thiophenylcarbonyl oder einen über das Stickstoffatom gebundenen Imidorest einer Dicarbonsäure,

B die direkte Bindung oder einen 1-4-gliedrigen Alkylen- oder Alkenylenrest der geradkettig oder verzweigt sein kann,

X Wasserstoff, Halogen, einen Cyan-, $C_1$-$C_4$-Alkyl-, Carboxyl-, Carbamoyl-, $C_1$-$C_4$-Alkylcarbonyl-, $C_1$-$C_4$-Alkoxycarbonyl- oder $C_1$-$C_4$-Alkylcarbamoylrest

m Null oder die Zahl 1 und

n Null oder die Zahl 1 oder 2 bedeuten.

Die Phenyl- und Naphthylreste Ar können ein- bis viermal durch Halogen oder durch Cyano-, Nitro-, $C_1$-$C_4$-Alkyl-, $C_1$-$C_4$-Halogenalkyl-, $C_1$-$C_4$-Alkoxy- durch Carboxyl-, Carbamoyl, $C_1$-$C_4$-Alkylcarbonyl-, $C_1$-$C_4$-Alkoxycarbonyl-, $C_1$-$C_4$-Alkylcarbamoyl- oder durch $C_1$-$C_4$-Alkylcarbamoyloxyreste aber auch einmal durch einen gegebenenfalls wie oben angegebenen substituierten Phenoxyrest substituiert sein. Unter 5-6-gliedrigen aromatischen oder benzannilierten Heterocyclen werden Pyrrol-, Furan-, Thiophen-, Pyridin-, Indol-, Benzofuran, Benzthiophen, Chinolin-, Pyrazin-, Oxazol-, Oxadiazol-, Benzoxazol, Thiazol, Thiadiazol-, Benzthiazolreste verstanden, die ihrerseits unsubstituiert oder wie oben substituiert sein können.

Unter Halogen werden Fluor, Chlor, Brom und Iod verstanden, vorzugsweise jedoch Fluor und Chlor.

Die Alkylreste umfassen in der angegebenen Kohlenstoffzahl auch alle möglichen Isomeren wie z.B. Methyl, Aethyl, n-Propyl-, Isopropyl, n-Butyl-, Isobutyl, sek.- und tert.-Butyl.

Dicarbonsäureamidoreste A sind die über das Stickstoffatom gebundenen Imide, beispielsweise der Phthalsäure, Malonsäure, Bernsteinsäure, Glutarsäure, Adipinsäure und Maleinsäure.

Unter den Verbindungen der Formel II sind diejenigen bevorzugt, in denen Ar einen unsubstituierten oder substituierten Phenyl-, Naphthyl- oder Thiadiazolrest bedeutet und m Null ist. Ferner diejenigen, in denen X die Cyanogruppe darstellt sowie diejenigen, in denen A ein Carbonylderivat wie z.B. einen $C_1$-$C_4$-Alkoxycarbonylrest, einen gegebenenfalls im Phenylring substituierten Benzoyl- oder Carbanilidorest, den Cyano-, Carbamoyl- oder einen $C_1$-$C_4$-Alkylcarbamoyl darstellt, wie beispielsweise diejenigen der Formel IIa

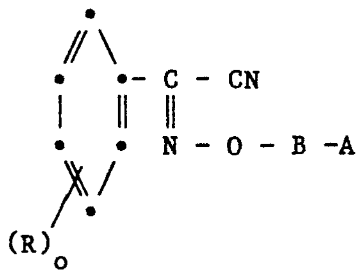

(IIa),

worin A und B die unter Formel I gegebene Bedeutung haben, während R Halogen, Cyan, Nitro, $C_1$-$C_4$-Alkyl, $C_1$-$C_4$-Halogenalkyl, $C_1$-$C_4$-Alkoxy, Carboxyl, Carbamoyl, $C_1$-$C_4$-Alkylcarbonyl, $C_1$-$C_4$-Alkoxycarbonyl, $C_1$-$C_4$-Alkylcarbamoyl oder $C_1$-$C_4$-Alkylcarbamoyloxy und o Null oder eine Zahl 1, 2, 3 oder 4 bedeutet, oder aber zwei benachbarte R die Butadienkette oder eines einen gegebenenfalls halogensubstituierten Phenoxyrest darstellen.

Von spezieller Bedeutung sind folgende Oxime der Formel II
Methylcarbamoyl-oximino-benzacetonitril,
2,2,2-Trifluoräthoxycarbonyl-oximino-benzacetonitril,
3-Trifluorcarbanilido-oximino-benzacetonitril,
(4-Chlorcarbanilido)-oximino-4-chlorbenzacetonitril,
(3,4-Dichlorcarbanilido)-oximino-4-tert.-butylbenzacetonitril,
Triphenylzinn-oximino-4-anisoylacetonitril,
Triphenylzinn-oximino-2,4-dichlorbenzacetonitril,
Bernsteinsäureimido-methoximino-benzacetonitril,
Methylcarbamoyl-oximino-(3,5-dibrom-2,4-dimethylcarbamoyloxy)-benzaldehyd,
Allyloxyäthoximino-α-naphthoyl-acetonitril,
Vinyloxyäthoximino-α-naphthoylacetonitril,
Methylthio-methoximino-4-anisoyl-acetonitril,
Isobutylcarbonyl-äthoximino-4-(2,4-dichlorphenoxy)-benzacetonitril,

ß-Aethoxy-α-(5-methoxy-1,3,4-thiadiazol-2-yl)-oximinooxalsäure-monoaldehyd,
4-Anisyloxy-methoximino-benzacetonitril,
Cyanomethoximino-2-fluorbenzacetonitril.

Die Oximäther der Formel II sind zum Schützen von Kulturpflanzen gegen die schädigende Wirkung von Sulfonylharnstoffen geeignet. Die Oximäther der Formel II können daher in Bezug auf ihre Anwendung in Kombination mit den vorgenannten Herbiziden als Gegenmittel, "Antidote" oder auch als "Safener" bezeichnet werden.

Ein solches Gegenmittel oder Antidote der Formel II kann je nach Anwendungszweck zur Vorbehandlung des Saatgutes der Kulturpflanze (Beizung des Samens oder der Stecklinge) eingesetzt oder vor oder nach der Saat in den Boden gegeben werden. Es kann aber auch für sich allein oder zusammen mit dem Herbizid vor oder nach dem Auflaufen der Pflanzen appliziert werden. Die Behandlung der Pflanze oder des Saatgutes mit dem Antidote kann daher grundsätzlich unabhängig vom Zeitpunkt der Applikation der phytotoxischen Chemikalien erfolgen. Die Behandlung der Pflanzen kann jedoch durch gleichzeitige Applikation von phythotoxischer Chemikalien und Gegenmittel (Tankmischung) erfolgen. Die preemergente Behandlung schliesst sowohl die Behandlung der Anbaufläche vor der Aussaat (ppi = pre plant incorporation) als auch die Behandlung der angesäten, aber noch nicht bewachsenen Anbauflächen ein.

Die Aufwandmengen des Gegenmittels im Verhältnis zum Herbizid richten sich weitgehend nach der Anwendungsart. Bei einer Feldbehandlung, bei der Herbizid und Gegenmittel entweder gleichzeitig (Tankmischung) oder separat appliziert werden, liegt das Verhältnis der Mengen von Gegenmittel zu Herbizid im Bereich von 1:10 bis 10:1. In der Regel wird bei einem Mengenverhältnis von Gegenmittel zu

Herbizid von 1:3 bis 3:1 die volle Schutzwirkung erreicht. Man ermittelt von Fall zu Fall, d.h. je nach verwendetem Herbizid-Typ, welches Verhältnis in bezug auf optimale Wirkung bei der speziellen Kulturpflanze das geeignetste ist. Bei der Samenbeizung und ähnlichen gezielten Schutzmassnahmen werden jedoch weit geringere Mengen Gegenmittel im Vergleich mit den später pro Hektar Anbaufläche verwendeten Mengen an Herbizid benötigt. Im allgemeinen werden bei der Samenbeizung pro kg Samen 0,5 - 50 g Gegenmittel benötigt. In der Regel wird mit 0,5 -4 g Gegenmittel pro kg Samen bereits die volle Schutzwirkung erreicht. Falls das Gegenmittel kurz vor der Aussaat durch Samenquellung appliziert werden soll, so werden zweckmässig Lösungen des Gegenmittels, welche den Wirkstoff in einer Konzentration von 1 - 10'000 ppm enthalten, verwendet. In der Regel wird mit Konzentrationen des Gegenmittels von 10 - 1000 ppm die volle Schutzwirkung erreicht.

In der Regel liegt zwischen protektiven Massnahmen, wie Samenbeizung und Behandlung von Stecklingen mit einem Gegenmittel der Formel II und der möglichen späteren Feldbehandlung mit Agrarchemikalien ein längerer Zeitraum. Vorbehandeltes Saat- und Pflanzengut kann später in Landwirtschaft, Gartenbau und Forstwirtschaft mit unterschiedlichen Chemikalien in Berührung kommen. Die Erfindung bezieht sich daher auch auf protektive Mittel für Kulturpflanzen, die als Wirkstoff ein Gegenmittel der Formel II zusammen mit üblichen Trägerstoffen enthalten. Solche Mittel können gegebenenfalls zusätzlich jene Agrarchemikalien enthalten, vor deren Einfluss die Kulturpflanze geschützt werden soll.

Als Kulturpflanzen gelten im Rahmen vorliegender Erfindung alle Pflanzen, die in irgendeiner Form Ertragsstoffe, wie Samen, Wurzeln, Stengel, Knollen, Blätter, Blüten, ferner Inhaltsstoffe, wie Oele, Zucker, Stärke, Eiweiss usw., produzieren und zu diesem Zweck

angebaut werden. Zu diesen Pflanzen gehören beispielsweise Getreidearten, wie Weizen, Roggen, Gerste und Hafer, daneben vor allem Reis, Sorghum, Mais, Baumwolle, Zuckerrüben, Zuckerrohr, Soja, Bohnen, und Erbsen.

Das Gegenmittel kann überall dort eingesetzt werden, wo eine Kulturpflanze der vorgenannten Art vor der schädlichen Wirkung von Sulfonylharnstoffen der Formel I geschützt werden soll.

Zur Beizung der Samen der Kulturpflanze werden die Samen mit dem Antidot der Formel II im gewünschten Mengenverhältnis gründlich vermischt.

Zur Verwendung werden die Verbindungen der Formel I und II in unveränderter Form oder vorzugsweise zusammen mit den in der Formulierungstechnik üblichen Hilfsmitteln eingesetzt und daher z.B. zu Emulsionskonzentraten, direkt versprühbaren und verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und II und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Di-octylphthalat, aliphatische Kohlenwasserstoffe wie Cyclohexan oder Paraffine, Alkohole und Glykole sowie deren Aether und Ester, wie Aethanol, Aethylenglykol, Aethylenglykolmonomethyl- oder -äthyläther, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I und II nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen wie wasserlösliche synthetische oberflächenaktive Verbindungen sein.

Als Seifen sind z.B. die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyltaurinsalze zu erwähnen.

Häufiger werden jedoch sog. synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali oder gegebenenfalls substituierte Ammoniumsalze vor und weisen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxyd-Addukten. Die sulfonierten Benzimidazolderivate enthalten vorzugsweise 2 Sulfonsäuregruppen und einen Fettsäurerest mit 8-22 C-Atomen. Alkylarylsulfonate sind z.B. die Na-, Ca- oder Triäthanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Aethylenoxyd-Adduktes oder Phospholipide in Frage.

Als nichtionisches Tensid kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglycoläther, Polypropylen-Polyäthylenoxyaddukte, Tributylphenoxypolyäthoxyäthanol, Polyäthylenglykol und Octylphenoxypolyäthanol erwähnt.

Ferner kommen auch Fettsäureester von Polyoxyäthylensorbitan wie das Polyoxyäthylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyldi(2-chloräthyl)äthylammoniumbromid.

Die in der Formulierungstechnik gebräuchlichen Tenside sind u.a. in folgenden Publikationen beschrieben:

"Mc Cutcheon's Detergents and Emulsifiers Annual"
MC Publishing Corp., Ridgewood, New Jersey, 1979
M. und J. Ash, "Encyclopedia of Surfactants", Vol, I-III
Chemical Publishing Co. Inc. New York 1981-1982
H. Stache "Tensid Taschenbuch" 2. Auflage,
C. Hanser Verlag, München und Wien 1981.

Diese Zubereitungen enthalten in der Regel 0,1 bis 99 %, insbesondere 0,1 bis 95 %, Wirkstoff der Formel I, 1 bis 99 % eines festen oder flüssigen Zusatzstoffes und 0 bis 25 %, insbesondere 0,1 bis 25 % eines Tensides.

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden. Die Aufwandmengen betragen in der Regel 0,1 bis 10 kg AS/ha, vorzugsweise 0,25 bis 5 kg AS/ha.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die Herstellung der Wirksubstanzen der Formel I und II kann nach bekannten Methoden erfolgen.

Verbindungen der Formel I werden in einem inerten, organischen Lösungsmittel hergestellt.

Nach einem ersten Verfahren werden die Verbindungen der Formel I erhalten, indem man ein Phenylsulfonamid der Formel III

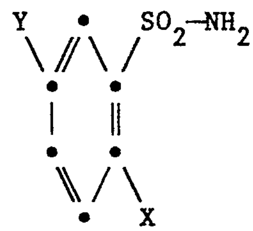

(III),

worin X und Y die unter Formel I gegebene Bedeutung haben, in Gegenwart einer Base mit einem N-Pyrimidinyl- oder -Triazinylcarbamat der Formel IV

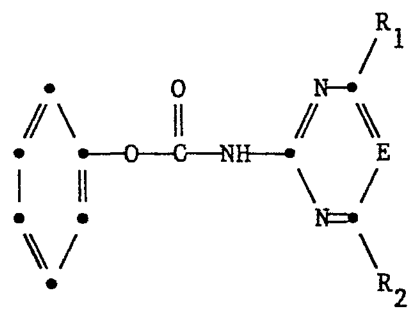

(IV),

worin E, $R_1$, $R_2$ die unter Formel I gegebene Bedeutung haben, umsetzt.

Nach einem zweiten Verfahren gelangt man zu Verbindungen der Formel I, indem man ein Phenylsulfonylisocyanat der Formel V

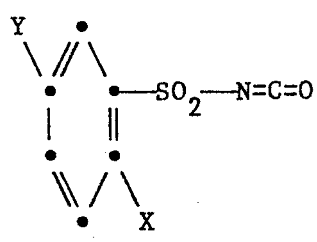

(V),

worin X und Y die unter Formel I gegebene Bedeutung haben, gegebenenfalls in Gegenwart einer Base, mit einem Amin der Formel VI

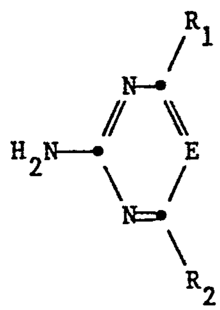

(VI),

worin E, $R_1$ und $R_2$ die unter Formel I gegebene Bedeutung haben, umsetzt.

Nach einem weiteren Verfahren werden Verbindungen der Formel I hergestellt, indem man ein Sulfonamid der oben angegebenen Formel III gegebenenfalls in Gegenwart einer Base mit einem Isocyanat oder Isothiocyanat der Formel VII

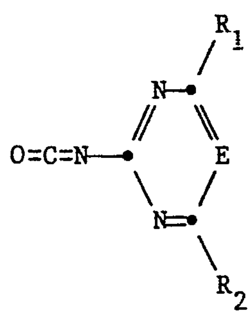

(VII),

worin E, $R_1$ und $R_2$ die unter Formel I gegebene Bedeutung haben, umsetzt.

Schliesslich kann man die Verbindungen der Formel I auch erhalten, indem man ein N-Phenylsulfonylcarbamat der Formel VIII

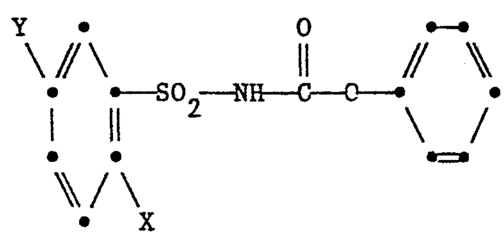

(VIII),

worin X und Y die unter Formel I gegebene Bedeutung haben, mit einem Amin der oben angegebenen Formel VI umsetzt.

Die erhaltenen Harnstoffe der Formel I können gewünschtenfalls mittels Aminen, Alkalimetall- oder Erdalkalimetallhydroxiden oder quaternären Ammoniumbasen in basische Additionsalze übergeführt werden. Dieses geschieht beispielsweise durch Umsetzen mit der äquimolaren Menge Base und Verdampfen des Lösungsmittels.

Die Ausgangsstoffe der Formeln III, V und VIII können nach folgenden Methoden hergestellt werden:

Die als Zwischenprodukte verwendeten Sulfonamide der Formel III werden aus den entsprechenden Anilinen durch Diazotierung und Austausch der Diazogruppe mit Schwefeldioxid in Gegenwart eines Katalysators wie Kupfer-I-chlorid in Salzsäure oder Essigsäure und Umsetzen des entstandenen Phenylsulfonylchlorids mit Ammoniumhydroxid-Lösung erhalten.

Die Verbindungen der Formel III können auch durch Sauerstoff-Alkylierung resp. Alkenylierung von Hydroxyphenyl-sulfonamiden mit den entsprechenden Halogeniden resp. Schwefelsäureestern erhalten werden.

Die Alkoxyphenylsulfonamide wiederum können aus den entsprechenden Alkoxyaniliden, wie bereits erwähnt oder durch Chlorsulfonylierung von Alkoxybenzolen und Umsatz der erhaltenen Phenylsulfonylchloriden mit Ammoniumhydroxy-Lösung gewonnen werden. Solche Reaktionen sind aus J. Am. Chem. Soc. 62, 603 (1940) bekannt geworden.

Die Phenylsulfonylisocyanate der Formel V können durch Umsetzungen der Sulfonamide der Formel III mit Phosgen in Anwesenheit von Butylisocyanat in einem chlorierten Kohlenwasserstoff als Lösungsmittel, bei Rückflusstemperatur erhalten werden. Aehnliche Darstellungen sind in "Newer Methods of Preparative Organic Chemistry" Band VI, 223-241, Academic Press New York und London, beschrieben.

Die N-Phenylsulfonylcarbamate der Formel VII werden durch Umsetzung der Sulfonamide der Formel II mit Diphenylcarbamat in Gegenwart einer Base erhalten. Aehnliche Verfahren sind in der japanischen Patentschrift 61 169 erwähnt.

Die Ausgangsmaterialien der Formeln IV, VI und VII sind bekannt oder können nach bekannten Methoden hergestellt werden.

Durch Umsetzung von Aminen der Formel VI mit Oxalylchlorid in chlorierten Kohlenwasserstoffen als Lösungsmittel, lassen sich Isocyanate der Formel VII herstellen. Amine der Formel VI sind bekannt und zum Teil im Handel erhältlich oder sie können nach bekannten Methoden hergestellt werden, siehe "The Chemistry of Heterocyclic Compounds" Band XIV, Interscience Publishers, New York, London.

Diese Umsetzungen zu Verbindungen der Formel I werden vorteilhafterweise in aprotischen, inerten, organischen Lösungsmitteln vorgenommen, wie Methylenchlorid, Tetrahydrofuran, Acetonitril, Dioxan, Toluol.

Die Reaktionstemperaturen liegen vorzugsweise zwischen -20° und +120°C. Die Umsetzungen verlaufen im allgemeinen leicht exotherm und können bei Raumtemperatur durchgeführt werden. Zwecks Abkürzung der Reaktionszeit oder auch zum Einleiten der Umsetzung wird zweckdienlich für kurze Zeit bis zum Siedepunkt des Reaktionsgemisches aufgewärmt. Die Reaktionszeiten können ebenfalls durch Zugabe einiger Tropfen Base oder Isocyanat als Reaktionskatalysator verkürzt werden.

Die Endprodukte können durch Einengen und/oder Verdampfen des Lösungsmittels isoliert und durch Umkristallisation oder Zerreiben des festen Rückstandes in Lösungsmitteln in denen sie sich nicht gut lösen, wie Aether, aromatischen Kohlenwasserstoffen oder chlorierten Kohlenwasserstoffen gereinigt werden.

Die Oximäther der Formel II werden hergestellt, indem man ein Salz eines Oxims der Formel IX

$$\begin{array}{l} Ar - (SO_n)_m - C - X \\ \qquad\qquad\qquad\quad \| \\ \qquad\qquad\qquad\quad N - OM \end{array} \qquad (IX)$$

worin M ein Alkalimetall oder Erdalkalimetall darstellt und Ar, X, m und n die unter Formel II gegebene Bedeutung haben, mit einem Halogenid der Formel X umsetzt

$$Hal - B - A \qquad (X),$$

worin A und B die unter Formel II gegebene Bedeutung haben.

Als Salze des Oxims der Formel IX sind, insbesondere die Natrium- und Kaliumsalze geeignet. Die Umsetzung des Oxims der Formel IX mit dem Halogenid der Formel X wird vorteilhaft in einem inerten organischen Lösungsmittel durchgeführt. Besonders geeignet sind polare Lösungsmittel, wie Acetonitril, Dimethylformamid und Dimethylsulfoxid, Dimethylacetamid, Methylpyrrolidon oder Tetramethylharnstoff. Die Reaktanten werden in der Regel in äquimolarer Menge eingesetzt. Es kann jedoch auch zum vollständigen Ablauf der

Reaktion der eine oder der andere Reaktionspartner im Ueberschuss eingesetzt werden. Die Umsetzung wird vorteilhaft bei erhöhter Temperatur, vorzugsweise zwischen 50 und 90°C, durchgeführt. Die Reaktion kann auch in einem anderen Lösungsmittel durchgeführt werden, wie beispielsweise Toluol, allerdings bei höherer Temperatur und längerer Wirkungsdauer.

Die Oxime der Formel II sind bekannte Verbindungen.

Als Herbizide werden in den folgenden Beispielen folgende Sulfonylharnstoffe geprüft:

A 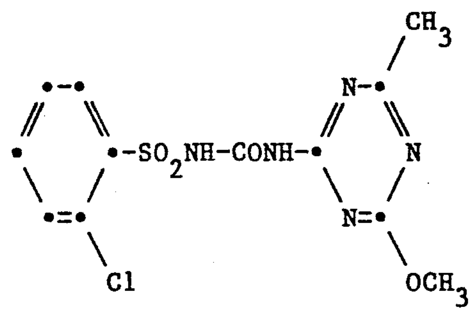 Smp. 174-176°

N-(2-Chlorphenylsulfonyl)-N'-(4-methoxy-6-methyltrizain-2-yl)-harnstoff.

B 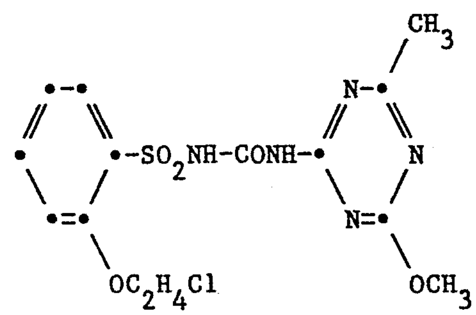 Smp. 174-175°

N-(2-ß-Chloräthoxyphenylsulfonyl)-N'-(4-methoxy-6-methyl-triazin-2-yl)-harnstoff.

C 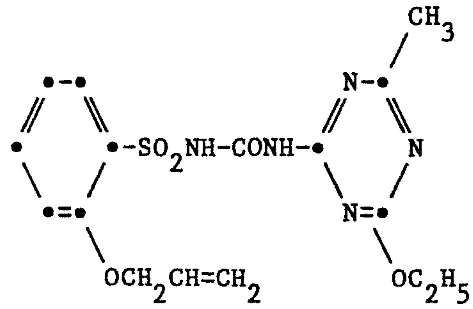 Smp. 129-131°

N-(2-Allyloxyphenylsulfonyl)-N'-(4-äthoxy-6-methyl-triazin-2-yl)-harnstoff.

D 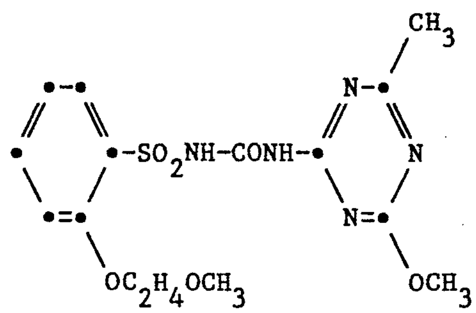 Smp. 134-138°

N-(2-Methoxyäthoxyphenylsulfonyl)-N'-(4-methoxy-6-methyl-triazin-2-yl)-harnstoff

E 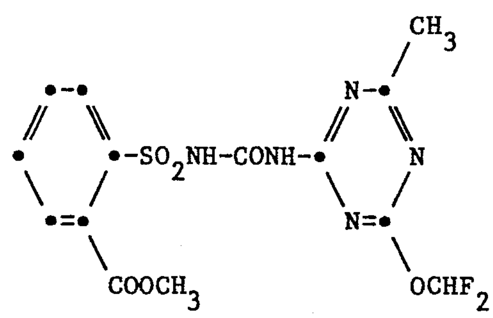 Smp. 163-164°

N-(2-Methylbenzoylsulfonyl)-N'-(4-difluormethoxy-6-methyl)-pyrimidin-2-yl)-harnstoff

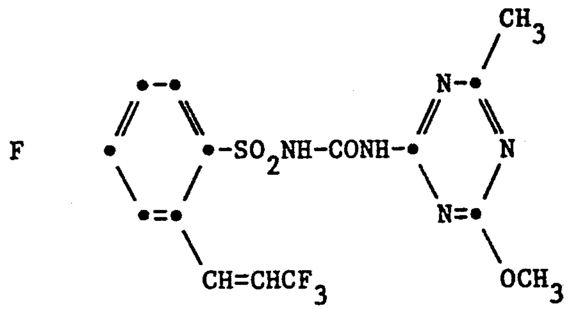

Smp. 159-160°

N-(2-γγγ-trifluoro-propyl-1-enylphenylsulfonyl)-N'-(4-methoxy-6-methyltriazin-2-yl)-harnstoff:

In den nachfolgenden Beispielen werden folgende Oxime der Formel II als Antidote geprüft:

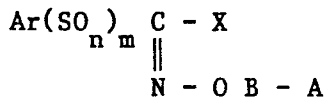

| No | Ar | $(SO_n)_m$ | X | B | A |
|---|---|---|---|---|---|
| 1 | 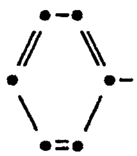 | - | CN | - | $CONHCH_3$ |
| 2 | 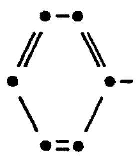 | - | CN | - | $COOCH_2CCl_3$ |
| 3 | 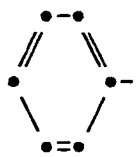 | - | CN | - | 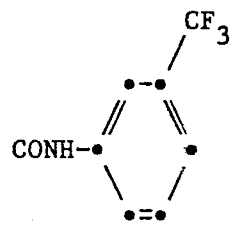 |
| 4 | 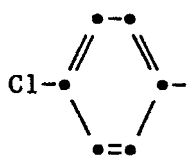 | - | CN | - | 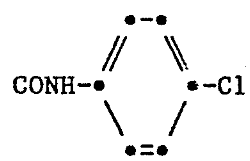 |
| 5 | 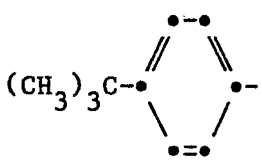 | - | CN | - | 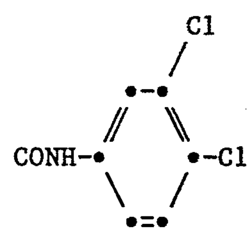 |
| 6 | 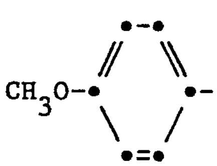 | - | CN | - | 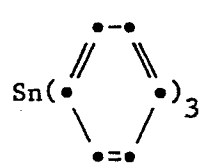 |

| No | Ar | $(SO_n)_m$ | X | B | A |
|---|---|---|---|---|---|
| 7 | 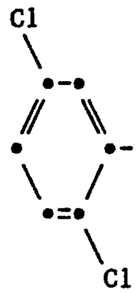 | - | CN | - | 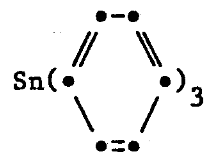 |
| 8 | 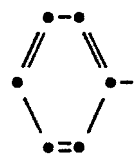 | - | CN | $CH_2$ | 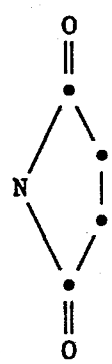 |
| 9 | 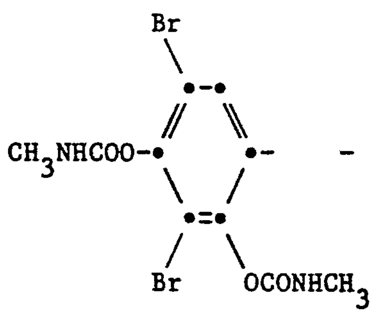 | - | H | - | $CONHCH_3$ |
| 10 | 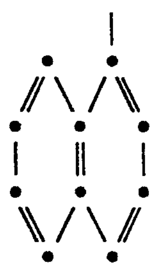 | - | CN | $C_2H_4$ | $OCH_2CH{=}CH_2$ |
| 11 | 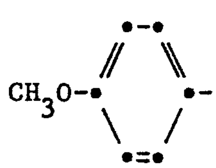 | - | CN | $CH_2$ | $SCH_3$ |

| No | Ar | $(SO_n)_m$ | X | B | A |
|---|---|---|---|---|---|
| 12 | 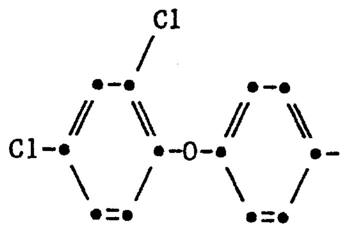 | - | CN | $CH(CH_3)$ | $COOC_4H_9$iso |
| 13 | 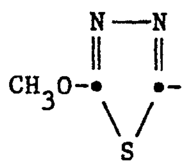 | - | $COOC_2H_5$ | - | H |
| 14 | 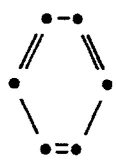 | - | CN | $CH_2$ | 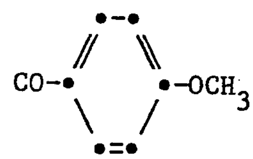 |
| 15 | 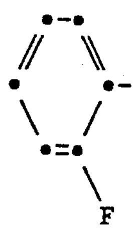 | - | CN | $CH_2$ | CN |
| 16 | 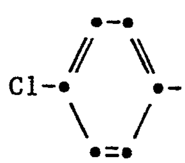 | - | CN | $CH_2$ | CN |
| 17 | 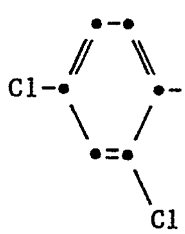 | - | CN | $CH_2$ | $CONH_2$ |

| No | Ar | $(SO_n)_m$ | X | B | A |
|---|---|---|---|---|---|
| 18 | 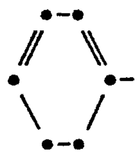 | $SO_2$ | CN | - | 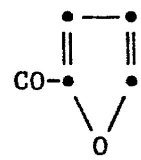 |
| 19 | 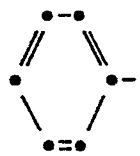 | $SO_2$ | CN | $-CH_2CH=CHCH_2-$ | H |

<u>Formulierungsbeispiele für Wirkstoffe der Formel II oder Mischungen</u>

a) <u>Spritzpulver</u>

| | a) | b) | c) |
|---|---|---|---|
| Wirkstoff der Formel II oder Mischung mit einem Herbizid der Formel I | 20 % | 60 % | 0,5 % |
| Na-Ligninsulfonat | 5 % | 5 % | 5 % |
| Na-Laurylsulfat | 3 % | - | - |
| Na-Diisobutylnaphthalinsulfonat | - | 6 % | 6 % |
| Octylphenolpolyäthylenglykoläther (7-8 Mol AeO) | - | 2 % | 2 % |
| Hochdisperse Kieselsäure | 5 % | 27 % | 27 % |
| Kaolin | 67 % | - | - |
| Natriumchlorid | - | - | 59,5 % |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

b) Emulsions-Konzentrat

| | a) | b) |
|---|---|---|
| Wirkstoff der Formel II oder Mischung mit einem Herbizid der Formel I | 10 % | 1 % |
| Octylphenolpolyäthylenglykoläther (4-5 Mol AeO) | 3 % | 3 % |
| Ca-Dodecylbenzolsulfonat | 3 % | 3 % |
| Ricinusölpolyglykoläther (36 Mol AeO) | 4 % | 4 % |
| Cyclohexanon | 30 % | 10 % |
| Xylolgemisch | 50 % | 79 % |

Aus diesem Konzentrat können durch Verdünnen mit Wasser Emulsionen jeder gewünschten Konzentration hergestellt werden.

c) Stäubemittel

| | a) | b) |
|---|---|---|
| Wirkstoff der Formel II oder Mischung mit einem Herbizid der Formel I | 0,1 % | 1 % |
| Talkum | 99,9 % | - |
| Kaolin | - | 99 % |

Man erhält anwendungsfertige Stäubemittel, indem der Wirkstoff mit dem Träger vermischt und auf einer geeigneten Mühle vermahlen wird.

d) Extruder Granulat

| | a) | b) |
|---|---|---|
| Wirkstoff der Formel II oder Mischung mit einem Herbizid der Formel I | 10 % | 1 % |
| Na-Ligninsulfonat | 2 % | 2 % |
| Carboxymethylcellulose | 1 % | 1 % |
| Kaolin | 87 % | 96 % |

Der Wirkstoff wird mit den Zusatzstoffen vermischt, vermahlen und mit Wasser angefeuchtet. Dieses Gemisch wird extrudiert und anschliessend im Luftstrom getrocknet.

e) Umhüllungs-Granulat

| | |
|---|---|
| Wirkstoff der Formel II oder Mischung mit einem Herbizid der Formel I | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

| f) Suspensions-Konzentrat | a) | b) |
|---|---|---|
| Wirkstoff der Formel II oder Mischung mit einem Herbizid | 40 % | 5 % |
| Aethylenglykol | 10 % | 10 % |
| Nonylphenolpolyäthylenglykoläther (15 Mol AeO) | 6 % | 1 % |
| Na-Ligninsulfonat | 10 % | 5 % |
| Carboxymethylcellulose | 1 % | 1 % |
| 37%ige wässrige Formaldehyd-Lösung | 0,2 % | 0,2 % |
| Silikonöl in Form einer 75%igen wässrigen Emulsion | 0,8 % | 0,8 % |
| Wasser | 32 % | 77 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

g) Salzlösung

| | |
|---|---|
| Wirkstoff der Formel II oder Mischung mit einem Herbizid der Formel I | 5 % |
| Isopropylamin | 1 % |
| Octylphenolpolyäthylenglykoläther (78 Mol AeO) | 3 % |
| Wasser | 91 % |

Biologische Beispiele

Die Fähigkeit der Verbindungen der Formel II, Kulturpflanzen vor der phytotoxischen Wirkung starker Herbizide zu schützen, kann aus den folgenden Beispielen ersehen werden. In den Versuchbeschreibungen werden die Verbindungen der Formel II als Antidote (Gegenmittel) bezeichnet.

Beispiel 1: Saatbeizung für Antidot; Herbizid Nachauflauf in Mais

Maissamen der Sorte "LG 5" werden mit der als Antidot zu prüfenden Substanz in einen Glasbehälter gemischt. Samen und Produkt werden durch Schütteln und Rotation gut zusammengemischt. Töpfe desselben Formats (oberer Ø 11 cm) werden mit Erde gefüllt und die gebeizten Samen werden eingesät. Nach dem Bedecken der Samen wird das Herbizid im Nachauflauf (Dreiblattstadium der Pflanze) appliziert. 21 Tage nach der Herbizidapplikation wird die Schutzwirkung des Antidot in Prozent bonitiert. Als Referenzen dienen dabei die mit Herbizid allein behandelten Pflanzen (keine Schutzwirkung) sowie die vollständig unbehandelte Kontrolle (100 % Schutzwirkung).

Die Resultate sind unten zusammengefasst:

| Herbizid No. | Herbizid Aufwandmenge | Antidote No | Antidote Aufwandmenge | relative Schutzwirkung |
|---|---|---|---|---|
| A | 31 g/ha | 15 | 1 g/kg Samen | 25 % |
| A | 31 g/ha | 15 | 0,5 g/kg Samen | 12.5 % |
| A | 15 g/ha | 15 | 1 g/kg Samen | 25 % |
| A | 15 g/ha | 15 | 0.5 g/kg Samen | 25 % |
| B | 125 g/ha | 15 | 1 g/kg Samen | 38 % |
| B | 125 g/ha | 15 | 0.5 g/kg Samen | 12.5 % |
| B | 125 g/ha | 15 | 0.25 g/kg Samen | 12.5 % |
| B | 62 g/ha | 15 | 1 g/kg Samen | 25 % |
| B | 62 g/ha | 15 | 0.5 g/kg Samen | 38 % |
| B | 62 g/ha | 15 | 0.25 g/kg Samen | 38 % |
| C | 125 g/ha | 15 | 1 g/kg Samen | 38 % |
| C | 125 g/ha | 15 | 0.5 g/kg Samen | 25 % |
| C | 62 g/ha | 15 | 1 g/kg Samen | 38 % |
| C | 62 g/ha | 15 | 0.5 g/kg Samen | 25 % |
| C | 62 g/ha | 15 | 0.25 g/kg Samen | 12.5 % |
| D | 125 g/ha | 15 | 1 g/kg Samen | 25 % |
| D | 125 g/ha | 15 | 0.5 g/kg Samen | 12 % |
| D | 125 g/ha | 15 | 0.25 g/kg Samen | 12 % |
| D | 62 g/ha | 15 | 1 g/kg Samen | 25 % |
| D | 62 g/ha | 15 | 0.5 g/kg Samen | 25 % |
| D | 62 g/ha | 15 | 0.25 g/kg Samen | 25 % |
| E | 125 g/ha | 15 | 1 g/kg Samen | 25 % |
| E | 62 g/ha | 15 | 1 g/kg Samen | 50 % |

| | | | | |
|---|---|---|---|---|
| E | 62 g/ha | 15 | 0.5 g/kg Samen | 12.5 % |
| E | 62 g/ha | 15 | 0.25 g/kg Samen | 12.5 % |
| E | 31 g/ha | 15 | 1 g/kg Samen | 38 % |
| E | 31 g/ha | 15 | 0.5 g/kg Samen | 25 % |

Beispiel 2: Saatbeizung für Antidot; Herbizid Vorauflauf in Mais

Maissamen der Sorte "LG 5" werden mit der als Antidot zu prüfenden Substanz in einen Glasbehälter gemischt. Samen und Produkt werden durch Schütteln und Rotation gut zusammengemischt. Töpfe desselben Formats (oberer ∅ 11 cm) werden mit Erde gefüllt und die gebeizten Samen werden eingesät. Nach dem Bedecken der Samen wird das Herbizid im Vorauflauf appliziert. 14 Tage nach der Herbizidapplikation wird die Schutzwirkung des Antidot in Prozent bonitiert. Als Referenzen dienen dabei die mit Herbizid allein behandelten Pflanzen (keine Schutzwirkung), sowie die vollständig unbehandelte Kontrolle (100 % Schutzwirkung).

Die Resultate sind unten zusammengefasst:

| Herbizid No | Aufwandmenge | Antidote No | Aufwandmenge | relative Schutzwirkung |
|---|---|---|---|---|
| A | 62 g/ha | 15 | 1 g/kg Samen | 12.5 % |
| A | 62 g/ha | 15 | 0.5 g/kg Samen | 12.5 % |
| A | 62 g/ha | 15 | 0.25 g/kg Samen | 12.5 % |
| A | 31 g/ha | 15 | 1 g/kg Samen | 25 % |
| A | 31 g/ha | 15 | 0.5 g/kg Samen | 25 % |
| A | 31 g/ha | 15 | 0.25 g/kg Samen | 12.5 % |
| A | 15 g/ha | 15 | 1 g/kg Samen | 12.5 % |
| A | 15 g/ha | 15 | 0.5 g/kg Samen | 25 % |
| A | 15 g/ha | 15 | 0.25 g/kg Samen | 25 % |

| | | | | | |
|---|---|---|---|---|---|
| C | 125 g/ha | 15 | 0.5 | g/kg Samen | 25 % |
| C | 125 g/ha | 15 | 0.25 | g/kg Samen | 38 % |
| | | | | | |
| D | 31 g/ha | 1 | 2 | g/kg Samen | 12.5 % |
| D | 31 g/ha | 2 | 2 | g/kg Samen | 12.5 % |
| D | 31 g/ha | 3 | 2 | g/kg Samen | 25 % |
| D | 31 g/ha | 4 | 2 | g/kg Samen | 12.5 % |
| D | 62 g/ha | 5 | 2 | g/kg Samen | 12.5 % |
| D | 31 g/ha | 6 | 2 | g/kg Samen | 12.5 % |
| D | 62 g/ha | 8 | 2 | g/kg Samen | 12.5 % |
| D | 62 g/ha | 9 | 2 | g/kg Samen | 12.5 % |
| D | 31 g/ha | 9 | 2 | g/kg Samen | 12.5 % |
| D | 62 g/ha | 13 | 2 | g/kg Samen | 25 % |
| D | 62 g/ha | 14 | 2 | g/kg Samen | 12.5 % |
| D | 125 g/ha | 15 | 1 | g/kg Samen | 12.5 % |
| D | 125 g/ha | 15 | 0.5 | g/kg Samen | 12.5 % |
| D | 125 g/ha | 15 | 0.25 | g/kg Samen | 12.5 % |
| D | 62 g/ha | 15 | 1 | g/kg Samen | 25 % |
| D | 62 g/ha | 15 | 0.5 | g/kg Samen | 25 % |
| D | 62 g/ha | 15 | 0.25 | g/kg Samen | 12.5 % |
| D | 31 g/ha | 15 | 1 | g/kg Samen | 38 % |
| D | 31 g/ha | 15 | 0.5 | g/kg Samen | 38 % |
| D | 31 g/ha | 15 | 0.25 | g/kg Samen | 38 % |

Beispiel 3: Saatbeizung für Antidot; Herbizid Vorauflauf in Sorghum

Sorghumsamen werden mit der als Antidot zu prüfenden Substanz in einen Glasbehälter gemischt. Samen und Produkt werden durch Schütteln und Rotation gut zusammengemischt. Töpfe desselben Formats (oberer Ø 11 cm) werden mit Erde gefüllt und die gebeizten Samen werden eingesät. Nach dem Bedecken der Samen wird das Herbizid im

Vorauflauf appliziert. 14 Tage nach der Herbizidapplikation wird die Schutzwirkung des Antidot in Prozent bonitiert. Als Referenzen dienen dabei die mit Herbizid allein behandelten Pflanzen (keine Schutzwirkung) sowie die vollständig unbehandelte Kontrolle (100 % Schutzwirkung).

Die Resultate sind unten zusammengefasst:

| Herbizid No | Herbizid Aufwandmenge | Antidote No | Antidote Aufwandmenge | relative Schutzwirkung |
|---|---|---|---|---|
| A | 125 g/ha | 15 | 1 g/kg Samen | 12.5 % |
| A | 125 g/ha | 15 | 0.5 g/kg Samen | 12.5 % |
| A | 125 g/ha | 15 | 0.25 g/kg Samen | 12.5 % |
| A | 62 g/ha | 15 | 1 g/kg Samen | 38 % |
| A | 62 g/ha | 15 | 0.5 g/kg Samen | 38 % |
| A | 62 g/ha | 15 | 0.25 g/kg Samen | 25 % |
| A | 31 g/ha | 15 | 1 g/kg Samen | 50 % |
| A | 31 g/ha | 15 | 0.5 g/kg Samen | 50 % |
| A | 31 g/ha | 15 | 0.25 g/kg Samen | 50 % |
| A | 15 g/ha | 15 | 1 g/kg Samen | 75 % |
| A | 15 g/ha | 15 | 0.5 g/kg Samen | 75 % |
| A | 15 g/ha | 15 | 0.25 g/kg Samen | 63 % |
| B | 125 g/ha | 15 | 0.5 g/kg Samen | 12.5 % |
| B | 125 g/ha | 15 | 0.25 g/kg Samen | 12.5 % |
| B | 62 g/ha | 15 | 1 g/kg Samen | 12.5 % |
| B | 62 g/ha | 15 | 0.5 g/kg Samen | 12.5 % |
| B | 62 g/ha | 15 | 0.25 g/kg Samen | 12.5 % |
| B | 31 g/ha | 15 | 1 g/kg Samen | 12.5 % |
| B | 31 g/ha | 15 | 0.5 g/kg Samen | 12.5 % |
| B | 31 g/ha | 15 | 0.25 g/kg Samen | 12.5 % |

| | | | | | |
|---|---|---|---|---|---|
| B | 15 g/ha | 15 | 1 | g/kg Samen | 38 % |
| B | 15 g/ha | 15 | 0.5 | g/kg Samen | 38 % |
| B | 15 g/ha | 15 | 0.25 | g/kg Samen | 38 % |
| | | | | | |
| C | 125 g/ha | 15 | 1 | g/kg Samen | 75 % |
| C | 125 g/ha | 15 | 0.5 | g/kg Samen | 75 % |
| C | 125 g/ha | 15 | 0.25 | g/kg Samen | 63 % |
| C | 62 g/ha | 15 | 1 | g/kg Samen | 63 % |
| C | 62 g/ha | 15 | 0.5 | g/kg Samen | 63 % |
| C | 62 g/ha | 15 | 0.25 | g/kg Samen | 50 % |
| C | 31 g/ha | 15 | 1 | g/kg Samen | 38 % |
| C | 31 g/ha | 15 | 0.5 | g/kg Samen | 38 % |
| C | 31 g/ha | 15 | 0.25 | g/kg Samen | 50 % |
| | | | | | |
| D | 125 g/ha | 15 | 1 | g/kg Samen | 12.5 % |
| D | 125 g/ha | 15 | 0.5 | g/kg Samen | 12.5 % |
| D | 125 g/ha | 15 | 0.25 | g/kg Samen | 12.5 % |
| D | 62 g/ha | 15 | 1 | g/kg Samen | 25 % |
| D | 62 g/ha | 15 | 0.5 | g/kg Samen | 25 % |
| D | 62 g/ha | 15 | 0.25 | g/kg Samen | 25 % |
| D | 31 g/ha | 15 | 1 | g/kg Samen | 50 % |
| D | 31 g/ha | 15 | 0.5 | g/kg Samen | 50 % |
| D | 31 g/ha | 15 | 0.25 | g/kg Samen | 25 % |
| D | 15 g/ha | 15 | 1 | g/kg Samen | 63 % |
| D | 15 g/ha | 15 | 0.5 | g/kg Samen | 63 % |
| D | 15 g/ha | 15 | 0.25 | g/kg Samen | 50 % |
| | | | | | |
| E | 15 g/ha | 15 | 1 | g/kg Samen | 12.5 % |
| E | 15 g/ha | 15 | 0.5 | g/kg Samen | 25 % |
| E | 15 g/ha | 15 | 0.25 | g/kg Samen | 25 % |

| | | | | |
|---|---|---|---|---|
| F | 125 g/ha | 15 | 1 g/kg Samen | 12.5 % |
| F | 125 g/ha | 15 | 0.5 g/kg Samen | 25 % |
| F | 125 g/ha | 15 | 0.25 g/kg Samen | 25 % |
| F | 62 g/ha | 15 | 1 g/kg Samen | 25 % |
| F | 62 g/ha | 15 | 0.5 g/kg Samen | 25 % |
| F | 62 g/ha | 15 | 0.25 g/kg Samen | 25 % |

Beispiel 4: Saatbeizung für Antidot, Herbizid Nachauflauf in Sorghum

Sorghumsamen der Sorte G 623 werden mit der als Safener zu prüfenden Substanz in einen Glasbehälter gemischt. Samen und Produkt werden durch Schütteln und Rotation gut zusammengemischt. Plastiktöpfe (oberer Ø 11 cm) werden mit Erde gefüllt und die gebeizten Samen werden eingesät. Nach dem Bedecken der Samen wird das Herbizid im Nachauflauf appliziert. 18 Tage nach der Herbizidapplikation wird die Schutzwirkung des Safeners in Prozent bonitiert. Als Referenz dienen dabei die mit Herbizid allein behandelten Pflanzen (keine Schutzwirkung) sowie die vollständig unbehandelte Kontrolle (100 % Schutzwirkung).

Die Resultate sind unten zusammengefasst:

| Herbizid No | Aufwandmenge | Antidote No | Aufwandmenge | relative Verbesserung der Schutzwirkung |
|---|---|---|---|---|
| A | 15 g/ha | 15 | 1 g/kg Samen | 38 % |
| A | 15 g/ha | 15 | 0.5 g/kg Samen | 25 % |
| A | 15 g/ha | 15 | 0.25 g/kg Samen | 12.5 % |
| C | 125 g/ha | 15 | 1 g/kg Samen | 25 % |
| C | 125 g/ha | 15 | 0.5 g/kg Samen | 12.5 % |
| C | 125 g/ha | 15 | 0.25 g/kg Samen | 12.5 % |

| | | | | |
|---|---|---|---|---|
| C | 62 g/ha | 15 | 1 g/kg Samen | 38 % |
| C | 62 g/ha | 15 | 0.5 g/kg Samen | 25 % |
| C | 62 g/ha | 15 | 0.25 g/kg Samen | 25 % |
| C | 31 g/ha | 15 | 1 g/kg Samen | 25 % |
| C | 31 g/ha | 15 | 0.5 g/kg Samen | 25 % |
| C | 31 g/ha | 15 | 0.25 g/kg Samen | 25 % |
| C | 15 g/ha | 15 | 1 g/kg Samen | 38 % |
| C | 15 g/ha | 15 | 0.5 g/kg Samen | 25 % |
| C | 15 g/ha | 15 | 0.25 g/kg Samen | 25 % |
| D | 62 g/ha | 15 | 1 g/kg Samen | 12.5 % |
| D | 62 g/ha | 15 | 0.5 g/kg Samen | 12.5 % |
| D | 62 g/ha | 15 | 0.25 g/kg Samen | 12.5 % |
| D | 31 g/ha | 15 | 1 g/kg Samen | 25 % |
| D | 31 g/ha | 15 | 0.5 g/kg Samen | 25 % |
| D | 31 g/ha | 15 | 0.25 g/kg Samen | 25 % |
| D | 15 g/ha | 15 | 1 g/kg Samen | 12.5 % |
| D | 15 g/ha | 15 | 0.5 g/kg Samen | 12.5 % |
| D | 15 g/ha | 15 | 0.25 g/kg Samen | 12.5 % |

## Beispiel 5: Tankmischung Vorauflauf in Gerste

Gerstensamen werden in Töpfe desselben Formats (oberer ∅ 11 cm), die 0,5 l Erde enthalten, im Gewächshaus ausgesät. Nach dem Bedecken der Samen wird die als Antidot zu prüfende Substanz zusammen mit dem Herbizid als Tankmischung auf die Bodenoberfläche appliziert. 21 Tage nach der Applikation wird die Schutzwirkung des Antidots in Prozent bonitiert. Als Referenzen dienen dabei die mit Herbizid allein behandelten Pflanzen (keine Schutzwirkung) sowie die vollständig unbehandelte Kontrolle (100 % Schutzwirkung).

Die Resultate sind unten zusammengefasst:

| Herbizid No | Herbizid Aufwandmenge | Antidote No | Antidote Aufwandmenge | relative Schutzwirkung |
|---|---|---|---|---|
| B | 62 g/ha | 1 | 62 g/kg Samen | 12.5 % |
| B | 62 g/ha | 3 | 62 g/kg Samen | 12.5 % |
| B | 125 g/ha | 4 | 125 g/kg Samen | 12.5 % |
| B | 62 g/ha | 4 | 62 g/kg Samen | 12.5 % |
| B | 125 g/ha | 5 | 125 g/kg Samen | 12.5 % |
| B | 62 g/ha | 5 | 62 g/kg Samen | 12.5 % |
| B | 125 g/ha | 7 | 125 g/kg Samen | 12.5 % |
| B | 62 g/ha | 7 | 62 g/kg Samen | 12.5 % |
| B | 62 g/ha | 8 | 62 g/kg Samen | 12.5 % |
| B | 125 g/ha | 10 | 125 g/kg Samen | 12.5 % |
| B | 62 g/ha | 10 | 62 g/kg Samen | 12.5 % |
| B | 62 g/ha | 11 | 62 g/kg Samen | 12.5 % |
| B | 62 g/ha | 12 | 62 g/kg Samen | 12.5 % |
| B | 62 g/ha | 13 | 62 g/kg Samen | 12.5 % |

Patentansprüche

1. Herbizides Mittel, das als herbiziden Wirkstoff einen Sulfonylharnstoff der Formel I

$$Q-SO_2-NH-CO-NH-T \qquad (I)$$

worin Q ein substituierter Phenylrest der Formel

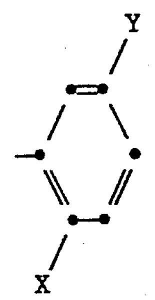

ist, worin X Halogen, $C_1$-$C_3$ Halogenalkenyl, $C_1$-$C_3$ Alkoxycarbonyl, $C_3$-$C_4$ Alkenyloxy, $C_1$-$C_3$ Halogenalkoxy, $C_1$-$C_3$ Alkoxy-$C_1$-$C_2$-alkoxy, Nitro, Y Wasserstoff oder Halogen bedeutet, T ein substituierter sechsgliedriger heterocyclischer Rest der Formel

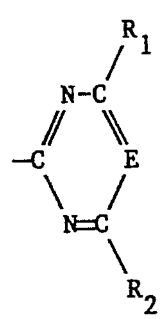

ist, worin E -CH= oder -N=, $R_1$ $C_1$-$C_3$ Alkyl, $C_1$-$C_3$ Alkoxy, $C_1$-$C_3$ Halogenalkoxy, $R_2$ $C_1$-$C_3$ Alkyl, $C_1$-$C_3$ Alkoxy bedeutet, enthält, dadurch gekennzeichnet, dass es als Antidot einen Oximäther der Formel II

0144283

$$\begin{array}{l} Ar(SO_n)_m - \underset{\displaystyle N - O - B - A}{\overset{\Vert}{C}} - X \end{array} \qquad (II)$$

worin Ar einen gegebenenfalls substituierten Phenyl-, Naphthyl- oder einen 5-6 gliedrigen aromatischen oder benzannelierten Heterocyclus mit 1-3 Heteroatomen,

A Wasserstoff, einen $C_1$-$C_4$-Alkoxy, $C_2$-$C_4$-Alkenyloxy, $C_1$-$C_4$-Alkylthio-, $C_2$-$C_4$-Alkenylthio-, Carboxyl-, Carbamoyl-, $C_1$-$C_4$-Alkylcarbonyl, $C_1$-$C_4$-Alkylcarbamoyl, $C_1$-$C_4$-Alkoxycarbonyl-, Benzoyl- und Carbanilidorest, wobei die Phenylringe unsubstituiert oder durch Halogen, Cyan, Nitro, $C_1$-$C_4$-Alkyl, $C_1$-$C_4$-Alkoxy oder $C_1$-$C_4$-Haloalkoxy substituiert sein können, sowie einen Furylcarbonyl-, Thiophencarbonyl- oder einen über das Stickstoffatom gebundenen Imidorest einer Dicarbonsäure,

B die direkte Bindung oder einen 1-4 gliedrigen Alkylen- oder Alkenylenrest, der geradkettig oder verzweigt sein kann,

X Wasserstoff, Halogen, einen $C_1$-$C_4$-Alkyl-, Cyan-, Carboxyl-, Carbamoyl-, $C_1$-$C_4$-Alkylcarbonyl-, $C_1$-$C_4$-Alkoxycarbonyl- oder $C_1$-$C_4$-Alkylcarbamoylrest,

m Null oder die Zahl 1 und

n Null oder die Zahl 1 oder 2 bedeuten, mit der Massgabe, dass, wenn Ar Phenyl, m Null und X Cyan bedeuten, der Rest -BA nicht Cyanomethyl sein darf, enthält.

2. Herbizides Mittel gemäss Anspruch 1, dadurch gekennzeichnet, dass das Mengenverhältnis vom Antidot zum herbiziden Wirkstoff zwischen 1:10 und 10:1 liegt.

3. Herbizides Mittel gemäss Anspruch 2, dadurch gekennzeichnet, dass das Mengenverhältnis vom Antidot zum herbiziden Wirkstoff zwischen 1:3 und 3:1 liegt.

4. Herbizides Mittel gemäss Anspruch 1, worin in der Formel I Y für Wasserstoff steht, dadurch gekennzeichnet, dass es als Antidot einen Oximäther der Formel II enthält.

5. Herbizides Mittel gemäss Anspruch 1, das als herbiziden Wirkstoff einen Sulfonylharnstoff, ausgewählt aus der Gruppe N-[2-(2'-Chloräthoxy)-phenyl-sulfonyl]-N'-(4-methyl-6-methoxy-triazin-2-yl)-harnstoff, N-[2-(2'-Methoxyäthoxy)-phenyl-sulfonyl]-N'-(4-methyl-6-methoxy-triazin-2-yl)-harnstoff, N-[2-(2-Butenyloxy)-phenyl-sulfonyl]-N'-(4-methyl-6-methoxy-triazin-2-yl)-harnstoff, N-[2-(3-Trifluor-propen-1-yl)-phenyl-sulfonyl]-N'-(4-methyl-6-methoxy-triazin-2-yl)-harnstoff, N-(2,5-Dichlorphenyl-sulfonyl)-N'-(4-methyl-6-methoxypyrimidin-2-yl)-harnstoff, N-(2-Methoxycarbonyl-phenyl-sulfonyl)-N'-(4-methyl-6-difluormethoxy-pyrimidin-2-yl)-harnstoff, N-(2-Pentafluoräthoxyphenyl-sulfonyl)-N'-(4,6-dimethoxy-triazin-2-yl)-harnstoff, N-(2-Chlorphenyl-sulfonyl)-N'-(4-methyl-6-methoxy-triazin-2-yl)-harnstoff, N-(2-Methoxycarbonyl-phenyl-sulfonyl)-N'-(4,6-dimethyl-pyrimidin-2-yl)-harnstoff, N-(2-Allyloxy-phenyl-sulfonyl)-N'-(4-methyl-6-äthoxy-triazin-2-yl)-harnstoff N-(2-Nitrophenyl-sulfonyl)-N'-(4-methyl-6-difluormethoxy-pyrimidin-2-yl)harnstoff und N-(2-Methoxycarbonyl-phenyl-sulfonyl)-N'-(4-methyl-6-methoxy-triazin-2-yl)-harnstoffenthält, dadurch gekennzeichnet, dass es als Antidot einen Oximäther der Formel II gemäss Anspruch 1 enthält.

6. Herbizides Mittel gemäss Anspruch 1, enthaltend eine herbizide Wirksubstanz der Formel I, dadurch gekennzeichnet, dass es als Antidot einen Oximäther der Formel II gemäss Anspruch 1, worin Ar einen unsubstituierten oder durch Halogen oder durch Cyano-, Nitro-, $C_1$-$C_4$-Alkyl, $C_1$-$C_4$-Halogenalkyl-, $C_1$-$C_4$-Alkoxy-, Carboxyl-, Carbamoyl-, $C_1$-$C_4$-Alkylcarbamoyl, $C_1$-$C_4$-Alkylcarbamoyloxyrest(e) substituierten Phenyl-, Naphthyl- oder Thiadiazolylrest oder einen durch einen gegebenenfalls wie oben substituierten Phenoxyrest substituierten Phenylrest,
X Wasserstoff, Cyan oder $C_1$-$C_4$-Alkoxycarbonyl,
B die direkte Bindung oder einen 1-4 gliedrigen Alkylen- oder Alkenylenrest der geradkettig oder verzweigt sein kann,
A Wasserstoff, Cyan, $C_1$-$C_4$-Alkoxy, $C_1$-$C_4$-Alklthio, $C_1$-$C_4$-Alkenyloxy, $C_1$-$C_4$-Alkoxcarbonyl, $C_1$-$C_4$-Halogenalkylcarbonyl, Benzoyl oder Carbanilido, wobei der Phenylrest durch Halogen, Cyan, Nitro, $C_1$-$C_4$-Alkyl, $C_1$-$C_4$-Halogenalkyl oder $C_1$-$C_4$-Alkoxy substituiert sein kann oder den Bernsteinsäureimidrest und
m Null bedeutet.

7. Herbizides Mittel gemäss Anspruch 1, enthaltend eine herbizide Wirksubstanz der Formel I, dadurch gekennzeichnet, dass es als Antidot einen Oximäther der Formel IIa

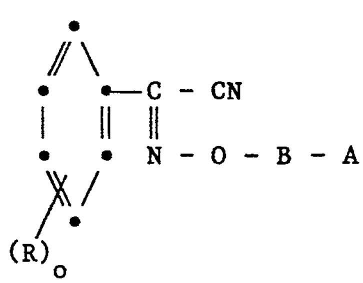

(IIa)

worin A und B die unter Formel I Anspruch 1 gegebene Bedeutung haben, während

R Halogen, Cyan, Nitro, $C_1$-$C_4$-Alkyl, $C_1$-$C_4$-Halogenalkyl, $C_1$-$C_4$-Alkoxy, Carboxyl, Carbamoyl, $C_1$-$C_4$-Alkylcarbonyl, $C_1$-$C_4$-Alkoxy-carbonyl, $C_1$-$C_4$-Alkylcarbamoyl, $C_1$-$C_4$-Alkylcarbamoyloxy und o Null oder eine Zahl 1, 2, 3 oder 4 bedeuten, oder aber zwei benachbarte R bilden die Butadienkette oder eines einen gegebenenfalls halogensubstituierten Phenoxyrest darstellen, mit der Massgabe dass wenn o Null ist, der Rest -BA nicht Cyanomethyl sein darf.

8. Herbizides Mittel gemäss Anspruch 1, enthaltend eine herbizide Wirksubstanz der Formel I, dadurch gekennzeichnet, dass es als Antidot einen Oximäther der Formel II gemäss Anspruch 1, ausgewählt aus

Methylcarbamoyl-oximino-benzacetonitril,
2,2,2-Trifluoräthoxycarbonyl-oximino-benzacetonitril,
3-Trifluorcarbanitido-oximino-benzacetonitril,
(4-Chlorcarbanilido)-oximino-4-chlorbenzacetonitril,
(3,4-Dichlorcarbanilido)-oximino-4-tert.-butylbenzacetonitril,
Triphenylzinn-oximino-4-anisoylacetonitril,
Triphenlzinn-oximino-2,4-dichlorbenzacetonitril,
Bernsteinsäureimidomethoximino-benzacetonitril,
Methylcarbamoyl-oximino-(3,5-dibrom-2,4-dimethylcarbamoyloxy)-benzaldehyd,
Allyloxyäthoximino-α-naphthoylacetonitril,
Vinyloxyäthoximino-α-naphthoylacetonitril,
Methylthiomethoximino-4-anisoylacetonitril,
Isobutyloxycarbonläth-1-oximino-4-(2,4-dichlorphenoxy)-benzacetonitril,
ß-Aethoxy-α-(5-methoxy-1,3,4-thiadiazol-2-yl)-oximino-oxalsäuremonoaldehyd,
4-Anisoylmethoximino-benzacetonitril,
Cyanomethoximino-2-fluorbenzacetonitril.

9. Herbizides Mittel gemäss Anspruch 1, enthaltend als Wirksubstanz der Formel I N-(2-Chlorphenylsulfonyl)-N'-(4-methoxy-6-methyl-triazin-2-yl)-harnstoff und als Antidot der Formel II Cyano-methoximino-2-fluorbenzacetonitril.

10. Herbizides Mittel gemäss Anspruch 1, enthaltend als Wirksubstanz der Formel I N-(2-ß-Chloräthylphenylsulfonyl)-N'-(4-methoxy-6-methyltriazin-2-yl)-harnstoff und als Antidot der Formel II Cyanomethoximino-2-fluorbenzacetoniril.

11. Herbizides Mittel gemäss Anspruch 1, enthaltend als Wirkstoff der Formel I N-(2-Allyloxyphenylsulfonyl)-N-(4-äthoxy-6-methyl-triazin-2-yl)-harnstoff und als Antidote der Formel II Cyano-methoximino-2-fluorbenzacetonitril

12. Herbizides Mittel gemäss Anspruch 1, enthaltend als Wirkstoff der Formel I N-(2-Methoxyäthoxyphenylsulfonyl)-N'-(4-methoxy-6-methyltriazin-2-yl)-harnstoff und als Antidote der Formel II Cyanomethoximino-2-fluorbenzacetonitril.

13. Herbizides Mittel gemäss Anspruch 1 enthaltend als Wirkstoff der Formel I N-(2-Methoxybenzoyl-sulfonyl)-N-(4-difluormethoxy-6-methyl-pyrimidin -2-yl)-harnstoff und als Antidote der Formel II Cyanomethoximino-2-fluorbenzacetonitril.

14. N-(2-γγγ-trifluoropropyl-1-enphenylsulfonyl)-N'-(4-methoxy-6-methylt riazin-2-yl)-harnstoff und als Antidote der Formel II Cyanomethoximino-2-fluorbenzacetonitril.

15. Verfahren zum Schützen von Pflanzenkulturen vor Schädigung durch Sulfonylharnstoffe, dadurch gekennzeichnet, dass man die Anwendung eines Sulfonylharnstoffs der Formel I gemäss Anspruch 1 auf die

Anbaufläche der Pflanzenkultur, auf das Saatgut der Pflanzen, oder auf die Pflanze selbst mit der Anwendung eines Oximäthers der Formel II gemäss Anspruch 1 kombiniert.

16. Verfahren gemäss Anspruch 15, dadurch gekennzeichnet, dass der Sulfonylharnstoff und das Antidot in einer Aufwandmenge von jeweils 0,005 bis 10 kg pro ha Kulturboden verwendet werden.

17. Verfahren gemäss Anspruch 15, dadurch gekennzeichnet, dass der Sulfonylharnstoff und das Antidot in einer Aufwandmenge von jeweils 0,05 bis 1 kg pro ha Kulturboden verwendet werden.

18. Verfahren gemäss Anspruch 15, dadurch gekennzeichnet, dass man einen Sulfonylharnstoff der Formel I verwendet, worin Y = H ist.

19. Verfahren gemäss Anspruch 15, dadurch gekennzeichnet, dass man die Anwendung eines Sulfonylharnstoffs ausgewählt aus der Gruppe N-[2-(2'-Chloräthoxy)-phenyl-sulfonyl]-N'-(4-methyl-6-methoxy-triazin-2-yl)-harnstoff, N-[2-(2'-Methoxyäthoxy)-phenyl-sulfonyl]-N'-(4-methyl-6-methoxy-triazin-2-yl)-harnstoff, N-[2-(2-Butenyloxy)-phenyl-sulfonyl]-N'-(4-methyl-6-methoxy-triazin-2-yl)-harnstoff, N-[2-(3-Trifluor-propen-1-yl)-phenyl-sulfonyl]-N'-(4-methyl-6-methoxy-triazin-2-yl)-harnstoff, N-(2,5-Dichlorphenyl-sulfonyl)-N'-(4-methyl-6-methoxypyrimidin-2-yl)-harnstoff, N-(2-Methoxycarbonyl-phenyl-sulfonyl)-N'-(4-methyl-6-difluormethoxy-pyrimidin-2-yl)-harnstoff, N-(2-Pentafluoräthoxyphenyl-sulfonyl)-N'-(4,6-dimethoxy-triazin-2-yl)-harnstoff, N-(2-Chlorphenyl-sulfonyl)-N'-(4-methyl-6-methoxy-triazin-2-yl)-harnstoff, N-(2-Methoxycarbonyl-phenyl-sulfonyl)-N'-(4,6-dimethyl-pyrimidin-2-yl)-harnstoff, N-(2-Allyloxy-phenyl-sulfonyl)-N'-(4-methyl-6-äthoxy-triazin-2-yl)-harnstoff N-(2-Nitrophenyl-sulfonyl)-N'-(4-methyl-6-difluormethoxy-pyrimidin-2-yl)harnstoff und N-(2-Methoxycarbonyl-phenyl-sulfonyl)-N'-(4-methyl-6-methoxy-triazin-2-yl)-harnstoff mit der Anwendung eines Oximäthers der Formel II gemäss Anspruch 1 kombiniert.

20. Verfahren gemäss Anspruch 15, dadurch gekennzeichnet, dass man die Anwendung eines Sulfonylharnstoffs der Formel I gemäss Anspruch 1 mit der Anwendung eines Oximäthers der Formel II, worin Ar einen unsubstituierten oder durch Halogen oder durch Cyano-, Nitro-, $C_1$-$C_4$-Alkyl, $C_1$-$C_4$-Halogenalkyl-, $C_1$-$C_4$-Alkoxy-, Carboxyl-, Carbamoyl-, $C_1$-$C_4$-Alkylcarbonyl-, $C_1$-$C_4$-Alkoxycarbonyl-, $C_1$-$C_4$-Alkylcarbamoyl- oder $C_1$-$C_4$-Alkylcarbamoyloxyrest(e) substituiertes Phenyl-, Naphthyl- oder Thiadiazolylrest oder einen durch einen gegebenenfalls wie oben substituierten Phenoxyrest substituierten Phenylrest,
X Wasserstoff, Cyan oder $C_1$-$C_4$-Alkoxycarbonyl,
B die direkte Bindung oder einen 1-4 gliedrigen Alkylen- oder Alkenylenrest, der geradkettig oder verzweigt sein kann,
A Wasserstoff, Cyan, $C_1$-$C_4$-Alkoxy, $C_1$-$C_4$-Alkylthio, $C_1$-$C_4$-Alkylenoxy, $C_1$-$C_4$-Alkoxycarbonyl, $C_1$-$C_4$-Halogenalkylcarbonyl, Benzoyl oder Carbanilido, wobei der Phenylrest durch Halogen, Cyan, Nitro, $C_1$-$C_4$-Alkyl, $C_1$-$C_4$-Halogenalkyl oder $C_1$-$C_4$-Alkoxy substituiert sein kann oder den Bernsteinsäurerest und
m Null bedeutet,
kombiniert.

21. Verfahren gemäss Anspruch 15, dadurch gekennzeichnet, dass man die Anwendung eines Sulfonylharnstoffes der Formel I mit der Anwendung eines Oximäthers der Formel IIa

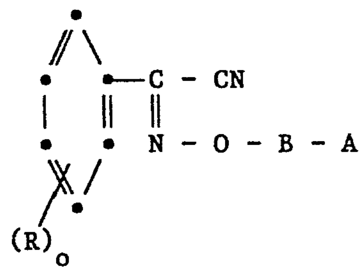

(IIa)

worin A und B die unter Formel I, Anspruch 1 gegebene Bedeutung haben, während

R Halogen, Cyan, Nitro, $C_1$-$C_4$-Alkyl, $C_1$-$C_4$-Halogenalkyl, $C_1$-$C_4$-Alkoxy, Carboxyl, Carbamoyl, $C_1$-$C_4$-Alkylcarbonyl, $C_1$-$C_4$-Alkoxycarbonyl, $C_1$-$C_4$-Alkylcarbamoyl, $C_1$-$C_4$-Alkoxycarbamoyloxy und

o Null oder eine Zahl 1, 2, 3 oder 4 bedeuten oder zwei benachbarte R auch die Butadienkette oder eines einen gegebenenfalls halogensubstituierten Phenoxyrest darstellt, mit der Massgabe, dass wenn o Null ist -B-A nicht Cyanomethyl sein darf,

kombiniert.

22. Verfahren gemäss Anspruch 15, dadurch gekennzeichnet, dass man die Anwendung eines Sulfonylharnstoffes der Formel I mit der Anwendung eines der folgenden Oximäther der Formel II kombiniert

Methylcarbamoyl-oximino-benzacetonitril,
2,2,2-Trifluoräthoxycarbonyl-oximino-benzacetonitril,
3-Trifluorcarbanilido-oximino-benzacetonitril,
(4-Chlorcarbanilido)-oximino-4-chlorbenzacetonitril,
(3,4-Dichlorcarbanilido)-oximino-4-tert.-butylbenzacetonitril,
Triphenylzinn-oximino-4-anisoylacetonitril,
Triphenylzinn-oximino-2,4-dichlorbenzacetonitril,
Bernsteinsäureimido-methoximino-benzacetonitril,

Methylcarbamoyl-oximino-(3,5-dibrom-2,4-dimethylcarbamoyloxy)-benzaldehyd,
Allyloxyäthoximino-α-naphthoyl-acetonitril,
Vinyloxyäthoximino-α-naphthoylacetonitril,
Methylthio-methoximino-4-anisoyl-acetonitril,
Isobutylcarbonyl-äthoximino-4-(2,4-dichlorphenoxy)-benzacetonitril,
ß-Aethoxy-α-(5-methoxy-1,3,4-thiadiazol-2-yl)-oximinooxalsäure-monoaldehyd, 4-Anisyloxy-methoximino-benzacetonitril,
Cyanomethoximino-2-fluorbenzacetonitril.

23. Vermehrungsgut von Kulturpflanzen, behandelt mit Cyanomethoxyimino-2-fluorbenzacetonitril.

24. Vermehrungsgut gemäss Anspruch 23, dadurch gekennzeichnet, dass 0,1 bis 10 g Cyanomethoxyimino-2-fluorbenzacetonitril pro kg Saatgut verwendet wird.

25. Vermehrungsgut gemäss Anspruch 23, dadurch gekennzeichnet, dass 0,25 bis 2 g Cyanomethoximino-2-fluorbenzacetonitril pro kg Saatgut verwendet wird.

26. Verfahren gemäss Anspruch 15 zum Schützen von Kulturpflanzen vor Schäden, die bei der Applikation von Sulfonylharnstoffen der Formel I gemäss Anspruch 1 auftreten, dadurch gekennzeichnet, dass man
a) die Anbaufläche für die Pflanze vor oder während der Applikation des Sulfonylharnstoffs oder
b) den Samen oder die Stecklinge der Pflanzen oder die Pflanze selbst mit einer wirksamen Menge eines Oximäthers der Formel II gemäss Anspruch 1 behandelt.

FO 7.5 NU/eg*

0144283

Europäisches Patentamt

**EUROPÄISCHER RECHERCHENBERICHT**

Nummer der Anmeldung

EP 84 81 0470

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
| Y,D | US-A-4 343 649 P.B. SWEETSER)<br>* Ansprüche *<br>--- | 1-22, 26 | A 01 N 47/36<br>A 01 N 25/32 |
| X | DE-A-2 808 317 (CIBA-GEIGY)<br>* Ansprüche 12-31 * | 23-25 | |
| Y | | 1-22, 26 | |
| | --- | | |
| Y | GB-A-2 028 797 (CIBA-GEIGY)<br>* Ansprüche; Seite 3, Zeilen 1-4 *<br>--- | 1-8,15 -22,26 | |
| Y | EP-A-0 010 058 (CIBA-GEIGY)<br>* Ansprüche; Seite 9, letzter Absatz *<br>--- | 1-8,15 -22,26 | RECHERCHIERTE SACHGEBIETE (Int. Cl.4)<br>A 01 N |
| Y | EP-A-0 010 143 (CIBA-GEIGY)<br>* Ansprüche; Seite 7, Zeilen 3-8 *<br>--- | 1-8,15 -22,26 | |
| Y | EP-A-0 012 158 (CIBA-GEIGY)<br>* Ansprüche; Seite 8d, letzter Absatz *<br>--- -/- | 1-8,15 -22,26 | |

Der vorliegende Recherchenbericht wurde für alle Patentansprüche erstellt.

| Recherchenort | Abschlußdatum der Recherche | Prüfer |
|---|---|---|
| DEN HAAG | 11-02-1985 | DECORTE D. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82

Europäisches Patentamt

**0144283**

Nummer der Anmeldung

# EUROPÄISCHER RECHERCHENBERICHT

EP 84 81 0470

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| P,X | CHEMICAL ABSTRACTS, Band 100, Nr. 15, 9. April 1984, Seite 189, Nr. 116361f, Columbus, Ohio, US; K.K. HATZIOS: "Interactions between selected herbicides and protectants on corn (Zea mays) & WEED SCI. 1983, 32(1), 51-8<br>* Zusammenfassung *<br>----- | 1-22, 26 | |
| | | | RECHERCHIERTE SACHGEBIETE (Int Cl 4) |

Der vorliegende Recherchenbericht wurde für alle Patentansprüche erstellt.

| Recherchenort | Abschlußdatum der Recherche | Prüfer |
|---|---|---|
| DEN HAAG | 11-02-1985 | DECORTE D. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument